# EUROPEAN PATENT SPECIFICATION

(11) **EP 1 839 644 B1**
(45) Date of publication and mention of the grant of the patent: **04.04.2018**
(21) Application number: 07003115.8
(22) Date of filing: 14.02.2007
(51) Int. Cl.: A61K 8/06, A61K 8/55, A61K 8/60, A61Q 19/00, A61K 8/86, B82Y 5/00

(54) **Nanoemulsion, production method thereof and cosmetic and dermatological composition containing it**
Nanoemulsion, ihr Herstellungsverfahren und kosmetische und dermatologische Zusammensetzung, die sie enthält
Nanoémulsion, procédé de fabrication correspondant et composition cosmétique et dermatologique la contenant

(30) Priority: 30.03.2006 BR PI0600941
(43) Date of publication of application: 03.10.2007
(73) Proprietor: Botica Comercial Farmacêutica Ltda, São José dos Pinhais PR 83065-260 (BR)
(72) Inventor: De Oliveira Praes, Carlos Eduardo, Uberaba Curitiba PR (BR); Cunha Motta, Fabrício, Cristo Rei Curitiba PR 80050-370 (BR); Feferman, Israel Henrique S., São José des Pinhais - PR 83065-260 (BR); Da Silva Belletti, Klézia Morais, Christo Rei Curitiba PR 80050-380 (BR); Krüger, Odivânia, Centro - Curitiba PR 80410-151 (BR); Patriarca, Paola Maria, Christo Rei - Curitiba 80050-450 (BR); Oliveira Mendes, Tania Regina, São José dos Pinhais - PR 83005-090 (BR); Dakiw Piaceski, Angela, Uberaba, Curitiba - PR 81550-360 (BR); Mazer Sauerman, Ronit, Água Verde, Curitiba PR 80240-210 (BR)
(74) Representative: Winter, Brandl, Fürniss, Hübner, Röss, Kaiser, Polte - Partnerschaft mbB

(56) References cited:
- EP-A- 0 852 941
- EP-A- 0 956 851
- EP-A- 1 010 413
- EP-A- 1 120 102
- WO-A-96/37192
- DE-A1- 10 213 957
- KÓNYA M ET AL: "Study of the microstructure of oil/water creams with thermal and rheological methods" JOURNAL OF THERMAL ANALYSIS AND CALORIMETRY, KLUWER ACADEMIC PUBLISHERS, DO, vol. 73, no. 2, 1 August 2003 (2003-08-01), pages 623-632, XP019254708 ISSN: 1572-8943
- UENER M ET AL: "INFLUENCE OF SURFACTANTS ON THE PHYSICAL STABILITY OF SOLID LIQUID NANOPARTICLE (SLN) FORMULATIONS" PHARMAZIE, DIE, GOVI VERLAG, ESCHBORN, DE, vol. 59, no. 4, 1 April 2004 (2004-04-01), page 331/332, XP009064777 ISSN: 0031-7144
- DATABASE EPODOC EUROPEAN PATENT OFFICE, THE HAGUE, NL; KR 2002 0076874 A 11 October 2002 (2002-10-11), ENPRANI CO LTD: "LOW-IRRITANT AND HIGH-MOISTURIZATION COSMETIC COMPOSITION" XP002542602 & KR 2002 0076874 A (ENPRANI CO LTD [KR]) 11 October 2002 (2002-10-11)
- SCHAFER-KORTING ET AL: "Lipid nanoparticles for improved topical application of drugs for skin diseases" ADVANCED DRUG DELIVERY REVIEWS, ELSEVIER BV, AMSTERDAM, NL, vol. 59, no. 6, 10 July 2007 (2007-07-10), pages 427-443, XP022205270 ISSN: 0169-409X

## Description

### INVENTION FIELD

This invention refers to a nanoemulsion of oil on water type, based on an emulsifiers ternary system that is stable under different temperature and physical stress conditions, besides being compatible with a great variety of cosmetics ingredients. The production method thereof comprises emulsification and homogenization under cold high pressure steps that makes possible the incorporation of active oily ingredients sensitive to higher temperatures.

### BACKGROUND OF THE INVENTION

Nanoemulsions are emulsions of oil on water type, whose oil particles enveloped by one or more emulsifiers (mycelia) are finely dispersed, and the average size of the particles distributed in the medium are in the nanometric scale. Nanoemulsions are recognizably produced by passing one or more times through a high pressure homogenizer, able to mechanically break the oil phase in the presence of one or more emulsifiers, dispersing it in the aqueous phase. The nanoemulsions confer benefits to the cosmetic compositions that are hardly obtainable with conventional emulsions. They present finer aspect and texture that results in differentiated sensory perception, besides favoring the transport of the active ingredients to the skin and the hair.

The nanoemulsions are known in the state of art, and were originally composed by water, phospholipids and oils. However, in spite of its high compatibility with the skin, these nanoemulsions do not present the physical and chemical stability required for cosmetic and dermatological compositions, as they tend to form an oxidation product, present phase separations, in addition to an unwanted yellow color and intense odor.

In this area, there is a special interest in the development of nanoemulsions that aggregate the benefits of improving the texture and stability of the compositions, enhancing the distribution of active ingredients on the skin and hair, without, however compromising the color and odor of the final products. There is also the interest that the production method of the nanoemulsion makes possible to incorporate thermo-sensitive active ingredients, that is, ingredients sensitive to higher temperatures. Additionally, the cosmetic and dermatological nanoemulsion-containing compositions should not have allergenic potential or cause itching reactions, which would make its use unfeasible. To conjugate such characteristics is the art embedded in the development of this kind of invention.

Many teachings are known in the state of art related to the nanoemulsion, among others it can be distinguished the following:

The patent application WO02080864A1 describes new nanoemulsions which comprise a ternary system comprising at least one anionic surfactant, such as sulphides, acetates or phosphates; one binding surfactant, such as non-ionic, semi-polars or amphoterics; and one cationic surfactant such as fatty amine salts or quaternary ammonium salts; on a 1:1:1 ratio, resulting in a neutral load. The purpose is to obtain a translucent to clear carrier, water-resistant and substantive to skin and hair.

The patent application US2004/0076598A1 describes a nanoemulsion based on sugar fatty esters, which are solid at temperatures lower than 45 °C, such as surfactants, and application thereof in cosmetic, dermatological and ophthalmological products. The surfactants must be in a concentration between 0.2% to 15.0% of the nanoemulsion total weight. The ratio between the oily phase and the surfactants must be between 2 and 10. It may also contain at least an amphiphilic ionic lipid, selected from anionic, cationic and alkylsuphonic derivatives in concentrations between 0.01% and 5.0% of the nanoemulsion total weight. EP-A1-0956851 describes the use of nanodispersions as carrier for actives in cosmetic compositions.

The patent application US2003/0078238A1 describes a method for stabilizing lecithin-based nanoemulsions through tocopherol derivatives, as well as the application thereof on the skin. The stabilizer must be present in concentrations equivalent to 0.001% to 20% of the lecithin concentration.

The patent US6902737 protects a translucent nanoemulsion, the production method and use thereof in the cosmetic, dermatological and ophthalmological fields. The nanoemulsion comprises a ternary system of surfactants constituted by the mix of at least two anionics, wherein at least one of them is a sorbitan fatty ester with 8 to 100 units of ethylene oxide and at least one sorbitan fatty ester; and by at least one ionic surfactant selected from cetylphosphate salts or palmitoil sarcosinate salts.

The patent US6335022 refers to a nanoemulsion based on a surfactant solid at temperatures lower than 45°C selected from sorbitan fatty esters containing ethylene oxide or not; at least a oil with molecular weight higher than 400; and at least a amphiphilic ionic lipid selected from dicetyl phosphate alkaline salts, cholesterol alkaline salts, lipoaminoacids, cationic lipids and alkylsulphonic derivatives. The ratio between the oily phase and the surfactants must be between 2 and 10.

Differently to this invention, the above mentioned patent applications and patents do not present the same combination of emulsifiers of this invention for obtaining of stable nanoemulsion. Also, this invention combines the benefits obtained by using low concentrations of the three emulsifiers, such as the high compatibility with the skin, the elimination of unwanted effects resulting from the use of lecithin in high concentrations and the cold emulsification method, which makes possible to incorporate active ingredients sensitive to high temperatures.

### DETAILED DESCRIPTION OF THE INVENTION

This invention describes a nanoemulsion to be used in cosmetic and dermatological compositions, as well as the production method thereof. Such nanoemulsion is obtained by combining three emulsifiers at specific proportions, which confers suitable physico-chemical stability and compatibility with a large variety of cosmetic ingredients, including ethanol and glycols, in addition to its cold production method making it possible to incorporate active ingredients sensitive to high temperatures.

The use of nanoemulsion according to this invention in cosmetic and dermatological compositions provides, in addition to a greater stability, a more homogeneous distribution of the active ingredients over the skin, the mucosa and the hair due the increased contact surface obtained with the reduced size of the mycelia. The reduced size of the mycelia can equally propitiate the delivery of the active ingredients to the deeper epidermal and capillary fiber layers, thereby increasing the efficacy of the compositions.

The emulsifying system of the nanoemulsion according to this invention comprises at least lecithin, PEG-40 Sorbitan Peroleate as fatty acid ester and condensed sorbitol with ethylene oxide, and a fatty acid diester and the product of condensation of methylglucose with polyglyceryl-3.

The nanoemulsion according to this invention is obtained through combining three emulsifiers. The first of them is the lecithin in an amount equivalent to 1% to 10% of the oily phase weight content, preferably between 2% and 6% of the oily phase content.

Since the nanoemulsions made only with the lecithin do not present a good physical (they present coalescence and phase separation under centrifugation and temperatures of 50°C) and chemical (they tend to oxidate and developing intense color and odor) stability, the nanoemulsions according to this invention associates a second non-ionic emulsifier, a fatty acid diester and the condensation product of methylglucose with polyglyceryl-3, preferably the methylglucose polyglyceryl-3 diestearate, in a concentration equivalent to 15% to 60% of the lecithin weight content, preferably from 30% to 40% of the lecithin weight content. The addition of this second emulsifier reduces the coalescence of the finely dispersed oil and lecithin mycelia, increasing the resistance thereof to weather at temperatures up to 50°C and to physical stress caused by centrifugation. This second emulsifier is in solid state at temperatures lower than 50°C and, therefore, the production method of emulsions containing such emulsifier occurs normally at higher temperatures (above 50-60°C) . Such fact would limit the production of nanoemulsions of active oily ingredients sensitive to higher temperatures, as is the case, for instance, of some vitamins.

To bypass such problem and to make possible to incorporate thermosensitive active ingredients, a third emulsifier was associated to the nanoemulsion, according to this invention, which prevents the fatty acid diester and the product of the condensation of methylglucose with polyglyceryl-3 to recrystalize in the mixture, allowing the production of the nanoemulsion at room temperature, usually lower than 30°C and compatible with the thermosensitive active ingredients. This third emulsifier is anionic, being the PEG-40 sorbitan peroleate, in concentration equivalent to 1 to 5 times the concentration value of the fatty acid diester and the product of the condensation of methylglucose with polyglyceryl-3, preferably from 2 to 3 times the concentration thereof.

The total concentration of the three emulsifiers blend must be between 1.3% and 46% of the total content of nanoemulsion oils, preferably between 3.8% and 15.6% of the total content of nanoemulsion oils. The combination of the three emulsifiers in the specific proportions according to this invention causes the necessary concentration of each one to become low enough to minimize the inconveniences of using each emulsifier separately, such as the fact of the lecithin developing unpleasant color and odor, and the requirement of a hot production method when a fatty acid diester and the product of the condensation of methylglucose with polyglyceryl-3 is used.

The table below represents the benefits obtained with the nanoemulsion prepared with the three emulsifiers according to the specific proportions set forth in this invention, comparatively to using individual emulsifiers:

| **Emulsifiers** | **Emulsification Process** | **Chemical stability** | **Centrifugation at 50°C/ Coalescence** | **Compatibility with the other cosmetic ingredients** |
|---|---|---|---|---|
| Lecithin | Cold | Low | Separation/ Yes | Low |
| Methylglucose distearate Polyglyceryl-3 | Hot | High | Separation/ Yes | Low |
| PEG-40 Sorbitan peroleate | Cold | High | Separation/ Yes | High |
| Lecithin + methylglucose polyglyceryl-3 distearate | Hot | High | Stable | High |
| Lecithin + methylglucose polyglyceryl-3 distearate + PEG-40 sorbitan peroleate | Cold | High | Stable | high |

Among the oils constituting the nanoemulsion there may be present active ingredients, thermosensitive or not, such as, for instance, vitamins A and K and derivatives thereof, as well as oily derivatives from other hydrosoluble vitamins, such as the esters of vitamin C. Other liposoluble active ingredients, such as, for instance, bisabolol or boldin diacetyl can also be present. Oily ingredients that act as emollients, lubricants, solubilizers, such as, for instance, cosmetic esters and esters, glycerides and derivatives, Guerbert alcohols, silicones, mineral oils, vegetable oils, fractions and derivatives thereof may be present acting as carriers and sensorial adjusters. Sunscreens, duly solubilized in their carriers can be present. The oily phase can also comprise antioxidant agents, conservatives, essential oils and fragrances. The total content of the oily phase can vary between 1.0% and 45.0% of the total weight of the nanoemulsion. The aqueous phase of the nanoemulsion may contain, in addition to water, glycols such as, for instance, glycerol, butylene glycol, propyleneglycol, hexylene glycols among others. It could also contain sugar, such as, for instance, glucose, fructose, galactose or xylitol among others. These compositions act as humectants, in addition to providing differentiated sensorial and assist in the solubilization of other components. They may contain conservative agents, such as parabenes, phenoxyethanol or benzyl alcohol. They may contain thickening agents, suspenders, filmmakers, such as, for instance, gums, acrylates, silicates, algae derivatives, carbomers, polymers with or without cross chains, cellulose derivatives, pure or modified starch, among others.

The nanoemulsion production method according to this invention initially involves mixing the non-thermosensitive oily phase components and PEG-40 Sorbitan Peroleate as fatty acid ester and condensed sorbitol with ethylene oxide, and fatty acid diester and the product of the condensation of methylglucose with polyglyceryl-3 in a container. Such mixture must be heated to a temperature higher than 50°C in order to occur fusion of the solid emulsifier. After that, upon cooling, the oily phase should be clear and homogeneous. After cooling at temperature lower than 30°C, add the lecithin and the active thermosensitive ingredients. Mix the oily phase with the aqueous phase and pass, at least once, through the high pressure homogenizer, with pressure between 50 Mpa and 200 Mpa.

The nanoemulsion produced according to the above described production method can be used as final product for application over the skin, mucosa and hair, or can also be incorporated in previously prepared cosmetic and dermatological compositions, acting as an additive. It can also be thickened and spiked after passing through the high pressure homogenizer. In all cases, the final cosmetic and dermatological compositions may contain, in addition to the described ingredients, fatty alcohols, waxes, resins, other emulsifiers and surfactants, ethoxilated or not, anionics or non-ionics, polymeric emulsifiers, alcohols, active hydrossoluble ingredients, such as vitamins and vegetable extracts, sunscreens, pigments, dyes, powders, among other formulation adjuvants.

The use of nanoemulsion according to this invention in cosmetic and dermatological compositions involves compositions for care, protection and makeup of skin, mucosa, scalp and hair. This nanoemulsion, at its purest condition, or incorporated to cosmetic and dermatological compositions upon application on the skin, mucosa and hairs promotes the homogeneous distribution of the ingredients on the surfaces, improving the performance and texture of the compositions.

The following examples are illustrative of the invention. The materials are mixed and the amounts are represented in weight percentage, based on the total weight of the composition.

### Example 1: Body Moisturiser Nanoemulsion

| **Composition** | **% m/m** |
|---|---|
| Lecithin | 0.60* |
| Methylglucose Polyglyceryl-3 Distearate | 0.24* |
| PEG-40 Sorbitan Peroleate | 0.72* |
| Emollients (octhyl estearate, capric/caprilic acid triglicerydes, dicaprilic ether) | 9.00* |
| Active thermosensitive oily ingredients (Derivatives of Vitamin A) | 1.00* |
| Conservatives (methylparabene, phenoxyethanol or others) | 0.50 |
| Sequestrant (EDTA dissodium) | 0.10 |
| Hydrossoluble dyeing substance | 0.01 |
| Thickeners (Hydroxiethylcelullose or others) | 1.00 |
| Humectants (glycerol, propyleneglycol) | 5.00 |
| Antioxidant (BHT, BHA among others) | 0.05* |
| Fragrance | 0.80 |
| Water | q.s.p* 100% |

| | |
|---|---|
| * In the example it is possible to notice that the content of Lecithin is equivalent to 6% of the oily phase weight content; that the Methylglucose Polyglyceryl-3 Distearate content is equivalent to 40% of the lecithin weight content; that the PEG-40 sorbitan peroleate content is equivalent to 3 times the Methylglucose polyglyceryl-3 distearate weight content; that the total mixture of emulsifiers is equivalent to 15.6% in weight of the total content of oils of the composition; and that the oily phase concentration is equivalent in weight to 10% of the total composition; according to detailed description of the invention. | |

Production method: Disperse the hydrossoluble components in water. In another container, fuse the Methylglucose Polyglyceryl-3 Distearate in the presence of the PEG-40 Sorbitan Peroleate, of the emollients and antioxidants at temperatures between 50 and 60 °C. Cool the oily phase to a temperature lower than 30 °C and add the Vitamin A derivative, the fragrance and the lecithin. Mix the aqueous and oily phases and pass once through the high pressure homogenizer, under a pressure of 120 Mpa to obtain the nanoemulsion.

### Example 2: FPS8 Face Lotion with Vitamin Nanoemulsion

| | |
|---|---|
| **Composition** | **% m/m** |
| Lecithin | 0.20* |
| Methylglucose Polyglyceryl-3 Distearate | 0.06* |
| PEG-40 Sorbitan Peroleate | 0.12* |
| Emollients (triglycerids of capric/caprilic acid) | 4.00* |
| Active thermosensitive oily ingredients (Derivatives of Vitamins A and C) | 2.00* |
| Conservatives (methylparabene, phenoxyethanol or others) | 0.50 |
| Sequestrant (EDTA dissodium) | 0.10 |
| Inorganic sunscreen dispersed in oily carrier (titanium dioxide in alkyl benzoate C12-15) | 10.00 |
| Thickener /Emulsifier (acrylate/alkyl acrylates co-polymer C10-30) | 0.30 |
| pH Corrector (triethanolamine) | 0.30 |
| Humectants (glycerol, propylene glycol) | 5.00 |
| Antioxidants (BHT, BHA, among others) | 0.05* |
| Fragrance | 0.80 |
| Water | q.s.p* 100% |

| | |
|---|---|
| * In the example 2 it is possible to notice that the content of Lecithin is equivalent to 3.33% of the oily phase weight content; that the Methylglucose Polyglyceryl-3 Distearate content is equivalent to 30% of the Lecithin weight content; that the PEG-40 Sorbitan Peroleate content is equivalent to 2 times the Methylglucose Polyglyceryl-3 Distearate weight content; that the total mixture of emulsifiers is equivalent to 6.33% in weight of the total content of oils of the composition; and that the oily phase concentration is equivalent to 20.42% of the weight of the total nanoemulsion composition; according to detailed description of the invention. | |

Production method: Fuse the Methylglucose Polyglyceryl-3 Distearate in a container in the presence of the PEG-40 sorbitan peroleate, of the emollients and antioxidants at temperature between 50 and 60 °C. Cool the oily phase to a temperature lower than 30 °C and add the Vitamins A and C derivatives, and the lecithin. Mix the oily phase with 30% of the total water content of the composition. Pass through the high pressure homogenizer 2 times, under a pressure of 100 Mpa, to obtain the nanoemulsion. In another container, disperse the hydrossoluble compositions and the thickener/emulsifier in the remaining amount of water. Add the inorganic sunscreen dispersed in its carrier and the fragrance over the aqueous phase, stirring all the time. Neutralize the pH of this second mix into a range between 5.50 and 6.50. Add the nanoemulsion over the mixture.

### Example 3: Face Cream for Night Use with Vitamin Nanoemulsion

| **Composition** | **% m/m** |
|---|---|
| Lecithin | 0.30* |
| Methylglucose Polyglyceryl-3 Distearate | 0.10* |
| PEG-40 Sorbitan Peroleate | 0.20* |
| Emollients (dibutyl adipate, octyl stearate) | 10.00* |
| Active thermosensitive oily ingredients (Derivatives of Vitamins A and C) | 2.00* |
| Conservatives (methylparabene, phenoxyethanol or others) | 0.50 |
| Sequestrant (EDTA dissodium) | 0.10 |
| Thickener (fatty alcohols, silicone wax, fatty ester) | 3.00 |
| Emulsifiers (ethoxylate fatty alcohols with 20 moles of ethylene oxide) | 1.00 |
| Hydrossoluble active ingredients (vitamins, proteins, amino acids, vegetable extracts) | 3.00 |
| Humectants (glycerol, propylene glycol) | 5.00 |
| Antioxidants (BHT, BHA, among others) | 0.05* |
| Fragrance | 0.80 |
| Water | q.s.p* 100% |

| | |
|---|---|
| *In the example 3 it is possible to notice that the content of Lecithin is equivalent to 4.29% of the oily phase weight content; that the Methylglucose Polyglyceryl-3 Distearate content is equivalent to 33.33% of the Lecithin weight content; that the PEG-40 sorbitan peroleate content is equivalent to 2 times the Methylglucose Polyglyceryl-3 Distearate weight content; that the total mixture of emulsifiers is equivalent to 8.57% in weight of the total content of oils of the composition; and that the oily phase concentration is equivalent to 23.5% of the weight of the total composition; according to detailed description of the invention. | |

Production method: Fuse the Methylglucose Polyglyceryl-3 Distearate in a container in the presence of the PEG-40 sorbitan peroleate, of half of the total contents of emollients, at a temperature between 50 and 60 °C. Cool the oily phase to a temperature lower than 30 °C and add the Vitamins A and C derivatives, and the lecithin. Mix the oily phase with 30% of the total water content of the composition. Pass through the high-pressure homogenizer once, under a pressure of 150 Mpa, to obtain the nanoemulsion. In another container, disperse the hydrossoluble compositions in the remaining amount of water and heat up to 80 °C. In one supplementary container fuse the emulsifier, the thickeners, the antioxidants and the other half of the total content of the emollients up to a temperature of 80 °C. Emulsify both phases stirring all the time up to 80 °C. Cool up to 30 °C, add the fragrance, the hydrossoluble active ingredients and the nanoemulsion.

The present invention was described from a particular embodiment, specifically, as a better way of realization of the invention.

## Claims

1. NANOEMULSION **characterized by** comprising an oily phase dispersed in an aqueous phase, the oily phase containing a combination of the emulsifiers lecithin, fatty acid diester and the product of the condensation of methylglucose with polyglyceryl-3, PEG-40 Sorbitan Peroleate as fatty acid ester and condensed sorbitol with ethylene oxide; in a total concentration equivalent to 1.3% to 46.0% in mass of the total oil content of the composition.

2. NANOEMULSION, according to claim 1, **characterized in that** the total concentration of the emulsifiers is equivalent, preferably, to 3.7% to 15.6% in mass of the total oil content of the composition.

3. NANOEMULSION, according to the claim 1, **characterized in that** the lecithin is present in concentration equivalent to 1.0% to 10.0% of the total oil content of the composition.

4. NANOEMULSION, according to the claim 3, **characterized in that** the lecithin is present in concentration between 2.0% to 6.0% of the total oil content of the composition.

5. NANOEMULSION, according to the claim 1, **characterized in that** the fatty acid diester and the product of condensation of methylglucose with polyglyceryl-3 is present in concentration equivalent to 15% to 60% of the lecithin content of the composition.

6. NANOEMULSION, according to the claim 5, **characterized in that** the fatty acid diester and the product of condensation of methylglucose with polyglyceryl-3 is present in concentration equivalent to 30% to 40% of the lecithin content of the composition.

7. NANOEMULSION, according to the claim 1, **characterized in that** the fatty acid diester and the product of condensation of methylglucose with polyglyceryl-3 is Polyglyceryl-3 Methylglucose Distearate.

8. NANOEMULSION, according to the claim 1, **characterized in that** the PEG-40 Sorbitan Peroleate is present in concentration equivalent to 1 to 5 times the concentration of the fatty acid diester and the product of the condensation of methylglucose with polyglyceryl-3.

9. NANOEMULSION, according to the claim 8, **characterized in that** the PEG-40 Sorbitan Peroleate is present in concentration equivalent to 2 to 3 times the concentration of the fatty acid diester and the product of the condensation of methylglucose with polyglyceryl-3.

10. NANOEMULSION, according to the claim 1, **characterized by** comprising the oily phase equivalent to 1 % to 45% in weight of the nanoemulsion total weight.

11. NANOEMULSION, according to the claim 1, **characterized in that** the components of the oily phase additionally comprises active oily ingredients, cosmetic esters and ethers, glycerides and derivatives, Gerbert alcohols, silicones, mineral oils, vegetable oils, fractions and derivatives thereof, sunscreens, antioxidant agents, conservatives, essential oils and fragrances.

12. NANOEMULSION, according to the claim 1, **characterized by** comprising the complementary ingredients of the conservative, sequestrant, antioxidant, thickener, filmmaker, humectant, opacifier, polymer, alcohol, solubilizer, powder, active ingredient, sunscreen, dyers, pigment, sensorial modifier, fragrance, vegetable extract, stabilizer, surfactant, emulsifier classes, among others.

13. PRODUCTION METHOD OF THE NANOEMULSION **characterized by** comprising the preparation of the oily phase which comprises the mixture of the oils with the fatty acid diester and the product of the condensation of methylglucose with polyglyceryl-3 and with the PEG-40 Sorbitan Peroleate as fatty acid ester and condensed sorbitol with ethylene oxide, followed by heating up to a temperature higher than 50°C, cooling to a temperature lower than 30°C, for subsequent addition of the lecithin and the thermosensitive ingredients.

14. METHOD, according to claim 13, **characterized by** comprising to pass the mixture of the aqueous and oily phases through the high pressure homogenizer at least once, at a pressure of 50Mpa to 200Mpa.

15. COSMETIC AND DERMATOLOGICAL COMPOSITION FOR APPLICATION OVER THE SKIN, MUCOSA AND HAIR **characterized by** comprising the nanoemulsion of claim 1.

16. COSMETIC AND DERMATOLOGICAL COMPOSITION, according to claim 15, **characterized by** comprising the basic formulation with 1% to 100% in weight of the nanoemulsion of claim 1.

## Patentansprüche

1. Nanoemulsion, **dadurch gekennzeichnet, dass** die Nanoemulsion eine ölige Phase aufweist, die in einer wässrigen Phase dispergiert ist, wobei die ölige Phase eine Kombination aus den Emulgatoren Lecithin, Fettsäurediester und dem Kondensationsprodukt von Methylglukose mit Polyglyceryl-3, PEG-40 Sorbitanperoleat als Fettsäureester und kondensiertem Sorbitol mit Ethylenoxid enthält, in einer Gesamtkonzentration, die äquivalent ist zu 1,3% bis 46% (Masse) des gesamten Ölgehalts der Zusammensetzung.

2. Nanoemulsion nach Anspruch 1, **dadurch gekennzeichnet, dass** die Gesamtkonzentration der Emulgatoren bevorzugt äquivalent ist zu 3,7% bis 15,6% (Masse) des gesamten Ölgehalts der Zusammensetzung.

3. Nanoemulsion nach Anspruch 1, **dadurch gekennzeichnet, dass** das Lecithin in einer Konzentration vorliegt, die äquivalent ist zu 1,0% bis 10,0% des gesamten Ölgehalts der Zusammensetzung

4. Nanoemulsion nach Anspruch 3, **dadurch gekennzeichnet, dass** das Lecithin in einer Konzentration zwischen 2,0% bis 6,0% des gesamten Ölgehalts der Zusammensetzung vorliegt.

5. Nanoemulsion nach Anspruch 1, **dadurch gekennzeichnet, dass** der Fettsäurediester und das Kondensationsprodukt von Methylglukose mit Polyglyceryl-3 in einer Konzentration vorliegt, die äquivalent ist zu 15% bis 60% des Lecithingehalts der Zusammensetzung.

6. Nanoemulsion nach Anspruch 5, **dadurch gekennzeichnet, dass** der Fettsäurediester und das Kondensationsprodukt von Methylglukose mit Polyglyceryl-3 in einer Konzentration vorliegt, die äquivalent ist zu 30% bis 40% des Lecithingehalts der Zusammensetzung.

7. Nanoemulsion nach Anspruch 1, **dadurch gekennzeichnet, dass** der Fettsäurediester und das Kondensationsprodukt von Methylglukose mit Polyglyceryl-3 Polyglyceryl-3-Methylglukose-Distearat ist.

8. Nanoemulsion nach Anspruch 1, **dadurch gekennzeichnet, dass** das PEG-40 Sorbitanperoleat in einer Konzentration vorliegt, die äquivalent ist zu der 1- bis 5-fachen Konzentration des Fettsäurediesters und dem Kondensationsprodukt von Methylglukose mit Polyglyceryl-3.

9. Nanoemulsion nach Anspruch 8, **dadurch gekennzeichnet, dass** das PEG-40 Sorbitanperoleat in einer Konzentration vorliegt, die äquivalent ist zu der 2- bis 3-fachen Konzentration des Fettsäurediesters und dem Kondensationsprodukt von Methylglukose mit Polyglyceryl-3.

10. Nanoemulsion nach Anspruch 1, **dadurch gekennzeichnet, dass** die Nanoemulsion die Ölphase äquivalent zu 1% bis 45% (Gewicht) des Gesamtgewichts der Nanoemulsion aufweist.

11. Nanoemulsion nach Anspruch 1, **dadurch gekennzeichnet, dass** die Komponenten der Ölphase zusätzlich aktive ölige Inhaltsstoffe, kosmetische Ester und Ether, Glyceride und Derivate, Gerbert-Alkohole, Silikone, Mineralöle, Pflanzenöle, Fraktionen und Derivate dieser, Sonnenschutzmittel, Antioxidationsmittel, Konservierungsmittel, ätherische Öle und Duftstoffe aufweisen.

12. Nanoemulsion nach Anspruch 1, **dadurch gekennzeichnet, dass** die Nanoemulsion unter anderem ergänzende Inhaltsstoffe aus Konservierungsmitteln, Komplexbildnern, Antioxidationsmitteln, Verdickungsmitteln, Filmbildnern, Feuchthaltemitteln, Trübungsmitteln, Polymeren, Alkoholen, Lösungsmitteln, Pulvern, aktive Inhaltsstoffen, Sonnenschutzmitteln, Färbemitteln, Pigmenten, sensorischen Modifikatoren, Duftstoffen, Pflanzenextrakten, Stabilisatoren, Tensiden und Emulgatorenklassen aufweist.

13. Verfahren zur Herstellung der Nanoemulsion, **dadurch gekennzeichnet, dass** das Verfahren die Vorbereitung der öligen Phase umfasst, die die Mischung der Öle mit dem Fettsäureester und dem Kondensationsprodukt von Methylglukose mit Polyglyceryl-3 und mit PEG-40 Sorbitanperoleat als Fettsäureester und kondensiertem Sorbitol mit Ethylenoxid aufweist, gefolgt durch Erhitzen bis zu einer Temperatur höher als 50°C und Abkühlen auf eine Temperatur niedriger als 30°C zur nachfolgenden Zugabe des Lecithins und den temperaturempfindlichen Inhaltsstoffen.

14. Verfahren nach Anspruch 13, **dadurch gekennzeichnet dass** das Verfahren umfasst, die Mischung der wässrigen und öligen Phase mindestens einmal durch den Hochdruckhomogenisator bei einem Druck von 50Mpa bis 200Mpa durchlaufen zu lassen.

15. Kosmetische und dermatologische Zusammensetzung zur Anwendung auf der Haut, Schleimhaut und dem Haar, **dadurch gekennzeichnet, dass** die Zusammensetzung eine Nanoemulsion nach Anspruch 1 aufweist.

16. Kosmetische und dermatologische Zusammensetzung nach Anspruch 15, **dadurch gekennzeichnet, dass** die Zusammensetzung die Basisrezeptur der Nanoemulsion nach Anspruch 1 zu 1% bis 100% (Gewicht) aufweist.

## Revendications

1. Nanoémulsion **caractérisée en ce qu'**elle comprend une phase huileuse dispersée dans une phase aqueuse, la phase huileuse contenant une combinaison des émulsifiants lécithine, diester d'acide gras et le produit de la condensation du méthylglucose avec le polyglycéryl-3, le PEG-40 Sorbitan Peroléate comme ester d'acide gras et du sorbitol condensé avec de l'oxyde d'éthylène ; en une concentration totale équivalente à 1,3 % à 46,0 % en masse de la teneur totale en huile de la composition.

2. Nanoémulsion, selon la revendication 1, **caractérisée** en que la concentration totale des émulsifiants est équivalente, de préférence, à 3,7 % à 15,6 % en masse de la teneur totale en huile de la composition.

3. Nanoémulsion, selon la revendication 1, **caractérisée en ce que** la lécithine est présente en concentration équivalente à 1,0 % à 10,0 % de la teneur totale en huile de la composition.

4. Nanoémulsion, selon la revendication 3, **caractérisée en ce que** la lécithine est présente en une concentration entre 2,0 % à 6,0 % de la teneur totale en huile de la composition.

5. Nanoémulsion, selon la revendication 1, **caractérisée en ce que** le diester d'acide gras et le produit de condensation du méthylglucose avec le polyglycéryl-3 est présent en concentration équivalente à 15 % à 60 % de la teneur en lécithine de la composition.

6. Nanoémulsion, selon la revendication 5, **caractérisée en ce que** le diester d'acide gras et le produit de condensation du méthylglucose avec le polyglycéryl-3 est présent en concentration équivalente à 30 % à 40 % de la teneur en lécithine de la composition.

7. Nanoémulsion, selon la revendication 1, **caractérisée en ce que** le diester d'acide gras et le produit de condensation du méthylglucose avec le polyglycéryl-3 est le polyglycéryl-3 méthylglucose distéarate.

8. Nanoémulsion, selon la revendication 1, **caractérisée en ce que** le PEG-40 Sorbitane Peroléate est présent en concentration équivalente à 1 à 5 fois la concentration du diester d'acide gras et du produit de condensation du méthylglucose avec le polyglycéryl-3.

9. Nanoémulsion, selon la revendication 8, **caractérisée en ce que** le PEG-40 Sorbitane Peroléate est présent en concentration équivalente à 2 à 3 fois la concentration du diester d'acide gras et du produit de condensation du méthylglucose avec le polyglycéryl-3.

10. Nanoémulsion, selon la revendication 1, **caractérisée en ce qu'**elle comprend la phase huileuse équivalente à 1 % à 45 % en poids du poids total de la nanoémulsion.

11. Nanoémulsion, selon la revendication 1, **caractérisée en ce que** les composants de la phase huileuse comprennent en outre des ingrédients huileux actifs, des esters et des éthers cosmétiques, des glycérides et dérivées, des alcools Gerbert, des silicones, des huiles minérales, des huiles végétales, des fractions et dérivés de ceux-ci, des crèmes solaires, des agents antioxydants, des conservateurs, des huiles essentielles et des parfums.

12. Nanoémulsions, selon la revendication 1, **caractérisée en ce qu'**elle comprend les ingrédients complémentaires du conservateur, du séquestrant, de l'antioxydant, de l'épaississant, du faiseur de film, de l'humectant, de l'opacifiant, du polymère, de l'alcool, du solubilisant, de la poudre, du principe actif, de la crème solaire, des teintureries, du pigment, du modificateur sensoriel, du parfum, de l'extrait végétal, du stabilisant, du tensioactif, des classes d'émulsifiant, entre autres.

13. Procédé de production de la nanoémulsion **caractérisé en ce qu'**il comprend la préparation de la phase huileuse qui comprend le mélange des huiles avec le diester d'acide gras et le produit de la condensation de méthylglucose avec le polyglycérine-3 et avec le PEG-40 Sorbitane Peroléate comme ester d'acide gras et le sorbitol condensé avec l'oxyde d'éthylène, suivi du chauffage jusqu'à température supérieure à 50 °C, du refroidissement à une température inférieure à 30 °C, pour l'addition ultérieure de la lécithine et des ingrédients thermosensibles.

14. Procédé, selon la revendication 13, **caractérisé en ce qu'**il comprend le passage du mélange des phases aqueuse et huileuse à travers l'homogénéisateur à haute pression au moins une fois, à une pression de 50 MPa à 200 MPa.

15. Composition cosmétique et dermatologique pour l'application sur la peau, la muqueuse et les cheveux **caractérisée en ce qu'**elle comprend la nanoémulsion selon la revendication 1.

16. Composition cosmétique et dermatologique, selon la revendication 15, **caractérisée en ce qu'**elle comprend la formulation basique avec 1 % à 100 % en poids de la nanoémulsion selon la revendication 1.
